(19) Europäisches Patentamt
European Patent Office
Office européen des brevets

(11) **EP 4 365 587 A1**

(12) **DEMANDE DE BREVET EUROPEEN**

(43) Date de publication:
**08.05.2024 Bulletin 2024/19**

(21) Numéro de dépôt: **23207815.4**

(22) Date de dépôt: **04.11.2023**

(51) Classification Internationale des Brevets (IPC):
**G01N 33/00** (2006.01)     **G01N 27/18** (2006.01)
**G01N 25/18** (2006.01)

(52) Classification Coopérative des Brevets (CPC):
**G01N 33/0004; G01N 25/18; G01N 27/18**

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**
Etats d'extension désignés:
**BA**
Etats de validation désignés:
**KH MA MD TN**

(30) Priorité: **06.11.2022 FR 2211524**

(71) Demandeur: **Commissariat à l'énergie atomique
et aux énergies alternatives
75015 Paris (FR)**

(72) Inventeurs:
• **BOURLON, Bertrand
38054 Grenoble cedex 09 (FR)**
• **TEULLE, Alexandre
38054 Grenoble cedex 09 (FR)**

(74) Mandataire: **INNOV-GROUP
310, avenue Berthelot
69372 Lyon Cedex 08 (FR)**

(54) **PROCÉDÉ DE TRAITEMENT DE MESURES D'UN CAPTEUR À CONDUCTION THERMIQUE**

(57) Procédé d'estimation d'une concentration de $n$ espèces gazeuses dans un gaz (3), $n$ étant un entier supérieur ou égal à 2, le procédé mettant en oeuvre un capteur de gaz (1), le capteur de gaz :
- comportant un élément sensible (4) configuré pour être chauffé à une température;
- étant configuré pour porter l'élément sensible à différentes températures ($T_i$) et pour générer un signal de détection ($S_i$) à chaque température;
le procédé comportant:

- a) exposition de l'élément sensible au gaz et chauffage de l'élément sensible à au moins $n$ différentes températures ($T_i$);
- b) à chaque température, mesure d'un signal de détection ($S_i$) et transmission du signal de détection à une unité de traitement (10);
- c) à partir des signaux de détection respectivement obtenus à chaque température, estimation d'une concentration en chaque espèce gazeuse ($x_j$) ;

**Description**

## DOMAINE TECHNIQUE

Le domaine technique de l'invention est la mesure d'un gaz par un capteur à conduction thermique.

## ART ANTERIEUR

Les détecteurs de gaz par conductivité thermique (TCD - Thermal conductivity detectors) sont couramment utilisés pour déterminer une concentration d'une espèce gazeuse dans un gaz porteur. Ce type de capteur permet de détecter ou encore d'estimer la concentration d'une espèce gazeuse dont la conductivité thermique est différente du gaz porteur. Par exemple, dans l'air, certaines espèces gazeuses, comme le méthane, ou l'hydrogène, ou le dioxyde de carbone, ou le butane, présentent une conductivité thermique différente de celle de l'air.

Il est usuel d'utiliser un capteur de mesure, exposé au gaz analysé, et un capteur de référence, exposé à un gaz de référence dont la conductivité thermique est connue. Le gaz analysé est le gaz de référence dans lequel une espèce gazeuse est susceptible d'être présente. On détermine la température du capteur de mesure et la température du capteur de référence. Si la conductivité thermique du gaz analysé est supérieure à celle du gaz de référence, la température du capteur de mesure diminue. Si la conductivité thermique du gaz analysé est inférieure à celle du gaz de référence, la température du capteur de mesure augmente. Les variations de températures dépendent de la concentration de l'espèce gazeuse dans le gaz analysé.

Des exemples de capteurs de gaz de type TCD sont décrits dans EP3118611 B1.

La publication Udina S et al " A micromachined thermoelectric sensor for natural gas analysis : Multivariate calibration results", décrit une calibration d'un capteur de gaz TCD par une régression de type moindres carrés partiels. La calibration suppose un apprentissage de façon à calibrer chaque vecteur de régression. Un tel apprentissage suppose un nombre conséquents de mélange de calibration. Dans l'exemple donné dans cette publication, 20 mélanges ont été utilisés pour l'apprentissage, et 18 pour la validation, et cela pour quantifier 4 espèces gazeuses.

Les inventeurs proposent un capteur calibré de façon à pouvoir estimer simultanément les concentrations respectives d'au moins deux espèces gazeuses différentes. La calibration est plus simple à mettre en oeuvre qu'une régression de type moindre carrés partiels.

## EXPOSE DE L'INVENTION

Un premier objet de l'invention est un procédé d'estimation d'une concentration de $n$ espèces gazeuses dans un gaz, $n$ étant un entier supérieur ou égal à 2, le procédé mettant en oeuvre un capteur de gaz, le capteur de gaz :

- comportant un élément sensible, disposé au contact du gaz, et configuré pour être chauffé par une piste conductrice de chauffage, la température de l'élément sensible dépendant de la conductivité thermique du gaz et d'un courant électrique de chauffage traversant la piste conductrice de chauffage;
- comportant un circuit de mesure, générant un signal de détection dépendant d'une température de l'élément sensible;

le procédé comportant les étapes suivantes :

- a) exposition de l'élément sensible au gaz et chauffage de l'élément sensible de façon à atteindre $n$ différentes températures ;
- b) pour chaque température, mesure d'un signal de détection et transmission du signal de détection à une unité de traitement;
- c) à partir des signaux de détection respectivement obtenus à chaque température, estimation d'une concentration en chaque espèce gazeuse;
- le procédé étant tel que l'étape c) comporte, pour chaque espèce gazeuse, une application d'une fonction de calibration, la fonction de calibration comportant une combinaison linéaire de termes, chaque terme dépendant du signal de détection respectivement mesuré à chaque température, chaque terme étant pondéré par un facteur de pondération;
- chaque facteur de pondération est préalablement déterminé au cours d'une phase de calibration, au cours de laquelle le capteur de gaz, ou un capteur de référence, représentatif du capteur de gaz, est successivement exposé à $n+1$ ensembles de concentrations connues de chaque espèce gazeuse, l'élément sensible étant successivement porté aux $n$ températures lors de chaque exposition.

Selon une possibilité, l'élément sensible comporte une piste conductrice de détection, reliée au circuit de mesure. Le signal de détection dépend d'un courant de détection traversant la piste conductrice de détection. La piste conductrice de détection peut être confondue avec la piste conductrice de chauffage.

De façon usuelle, l'élément sensible est une membrane.

L'étape a) peut comporter une application de *n* différents courants prédéterminés à travers la piste conductrice de chauffage.

Chaque piste conductrice, de chauffage et/ou de détection, peut être formée par un fil ou une bande de matériau conducteur.

Selon une possibilité, la phase de calibration comporte :

- i) obtention d'un signal de détection lors de chaque exposition du capteur de gaz à un ensemble de concentrations connues de chaque espèce gazeuse ;
- ii) constitution d'une matrice à l'aide des signaux de détection obtenus lors de l'étape i) ;
- iii) pour chaque espèce, formation d'un vecteur, comportant les concentrations de l'espèce gazeuse dans chacun des $n+1$ ensembles de concentrations ;
- iv) calcul ou estimation d'un vecteur, de facteurs de pondération associés à chaque espèce gazeuse, ledit vecteur correspondant à un produit matriciel entre l'inverse de la matrice résultant de l'étape ii) et le vecteur résultant de l'étape iii).

$n$ peut être égal à 2.

Le gaz peut être de l'air ambiant, les espèces gazeuses mesurées étant le $CO_2$ et l'humidité. Selon une possibilité:

- au cours de la phase de calibration, la concentration de chaque espèce gazeuse varie entre une concentration minimale et une concentration maximale ;
- la fonction de calibration définie pour chaque espèce gazeuse est valide entre ladite concentration minimale et ladite concentration maximale.

Un deuxième objet de l'invention est un capteur de gaz, comportant un élément sensible:

- configuré pour être disposé au contact du gaz, et pour être chauffé par une piste conductrice de chauffage, la température de l'élément sensible dépendant de la conductivité thermique du gaz et d'un courant traversant la piste conductrice de chauffage;
- un circuit de mesure d'une température de l'élément sensible, ladite température dépendant de la conductivité thermique du gaz ;
- le capteur de gaz étant configuré pour porter l'élément sensible à différentes températures et pour générer un signal de détection à chaque température, le signal de détection étant représentatif de la conductivité thermique du gaz à ladite température;
- le capteur de gaz étant relié à une unité de traitement, configurée pour mettre en oeuvre l'étape c) d'un procédé selon le premier objet de l'invention.

L'élément sensible peut être une membrane. La piste conductrice de chauffage peut être un fil électriquement conducteur.

Le circuit de mesure de la température de l'élément sensible est un circuit de mesure de la résistance d'une piste électrique de détection traversant l'élément sensible.

Un troisième objet de l'invention est un système de mesure comportant plusieurs capteurs de gaz selon le deuxième objet de l'invention.

Selon une possibilité, au moins un capteur de gaz est exposé à un gaz de référence, dont la composition est connue, et au moins un capteur de gaz est exposé à un gaz dont la teneur en au moins une espèce gazeuse est inconnue.

Selon une possibilité, le système de mesure comporte quatre capteurs de gaz selon le deuxième objet de l'invention agencés en pont de Wheatstone, de telle sorte que deux capteurs opposés sont exposés au gaz de référence et deux autres capteurs opposés sont exposés au gaz dont la teneur en au moins une espèce gazeuse est inconnue.

L'invention sera mieux comprise à la lecture de l'exposé des exemples de réalisation présentés, dans la suite de la description, en lien avec les figures listées ci-dessous.

## FIGURES

La figure 1 représente un exemple de capteur selon l'invention.

La figure 2 schématise les principales étapes de traitement de signaux de détection résultant d'un capteur à conduction thermique.

La figure 3 schématise un montage expérimental.

La figure 4A montre une comparaison entre des mesures de CO= résultant d'un capteur à conduction thermique, en mettant en oeuvre un procédé selon l'invention, et des mesures de référence.

La figure 4B montre une comparaison entre des mesures d'humidité relative résultant d'un capteur à conduction thermique, en mettant en oeuvre un procédé selon l'invention, et des mesures de référence.

## EXPOSE DE MODES DE REALISATION PARTICULIERS

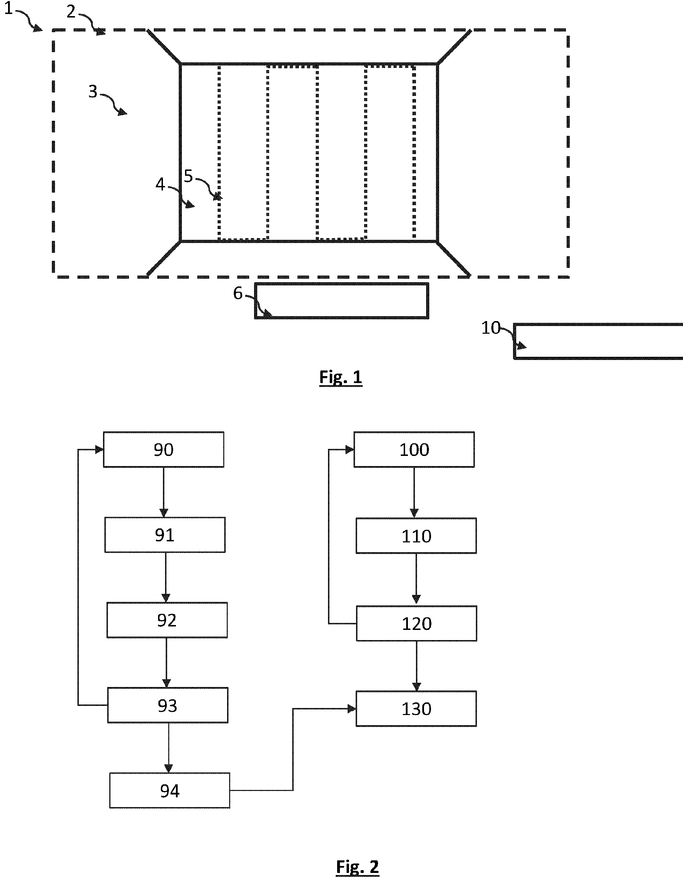

La figure 1 représente un exemple de capteur de gaz 1 à conductivité thermique. Le capteur de gaz comporte une enceinte 2, destinée à recevoir un gaz à analyser 3. L'enceinte comporte un élément sensible 4, destiné à être successivement chauffé à différentes températures $T_i$. Dans cet exemple, l'élément sensible 4 est une membrane suspendue, telle que décrite dans EP3118611 B1. La membrane suspendue est parcourue par un fil conducteur 5, de façon à pouvoir être portée à chaque température $T_i$. La membrane peut par exemple être formée de nitrure de silicium, et parcourue par un fil électriquement conducteur en platine. Le fil électriquement conducteur forme une piste conductrice de chauffage. Lorsqu'il est traversé par un courant électrique de chauffage $E_i$, la membrane est portée à une température $T_i$. La température $T_i$ de la membrane dépend du courant électrique et de la conductivité thermique du gaz. Le courant électrique de chauffage peut être commandé en intensité ou en tension.

L'élément sensible 4 est relié à un circuit de mesure de température 6. Le circuit de mesure de température peut être connecté à une piste conductrice de détection, traversant la membrane, de façon à estimer la température de la membrane par une mesure de thermo-résistance. La mesure de la résistance de la piste conductrice de détection est ainsi une indication indirecte de la conductivité thermique du gaz analysé.

La piste conductrice de détection peut être confondue avec la piste conductrice de chauffage et former une même piste.

Le principe de fonctionnement du capteur est basé sur une mesure d'une température $T_i$ de l'élément sensible 4 en présence d'un gaz, suite à l'application d'un courant électrique de chauffage. Pour chaque valeur du courant électrique de chauffage, la température $T_i$ dépend de la conductivité thermique du gaz comme décrit dans l'art antérieur. Par valeur d'un courant électrique de chauffage, on entend une valeur d'une intensité ou d'une tension du courant de chauffage.

Le gaz comporte au moins deux espèces gazeuses, portées par un gaz porteur, dont on cherche à déterminer la concentration. Dans l'exemple décrit par la suite, le gaz porteur est de l'air, et on cherche à estimer la concentration de la vapeur d'eau ainsi que du $CO_2$.

Le capteur est relié à une unité de traitement 10, configurée pour mettre en oeuvre certaines étapes décrites en lien avec la figure 2. L'unité de traitement est par exemple un microprocesseur relié à une mémoire comportant des instructions pour la mise en oeuvre desdites étapes.

Etape 100 : le gaz est admis dans l'enceinte, de façon à être mis au contact de l'élément sensible.

Etape 110 : l'élément sensible est porté à une température $T_i$ en appliquant un courant électrique de chauffage dont l'intensité ou la tension est connue. On mesure ensuite la température de l'élément sensible, par exemple en déterminant la résistance de la piste électrique de détection.. La température $T_i$ est déterminée par le circuit de mesure de température 6. Comme précédemment indiqué, la température peut être mesurée en mesurant la résistance de la piste conductrice de détection. Ainsi, le capteur de gaz génère un signal de détection $S_i$ représentatif de la conductivité thermique $\lambda_i$ du gaz à la température $T_i$.

Etape 120 : L'étape 110 est répétée pour atteindre deux températures $T_i$ différentes.

Etape 130 : Au cours de cette étape, les concentrations $x_j$ $(1 \leq j \leq 2)$ de chaque espèce gazeuses sont estimées en appliquant une fonction de calibration $f_j$, préalablement définie pour chaque espèce gazeuse, aux signaux de détection $S_i$

Ainsi, $x_j = f_j(S_i)$

Dans cet exemple :

$$x_1 = f_1(S_1, S_2) \ (1)$$

et

$$x_2 = f_2(S_1, S_2) \ (2)$$

Chaque fonction de calibration $f_j$, associée à un espèce gazeuse $x_j$, comporte une combinaison linéaire de termes, chaque terme dépendant d'un signal de détection $S_i$ mesuré à la température $T_i$.

Dans cet exemple, chaque fonction de calibration $f_j$ est telle que :

$$x_j = f_j(S_i) = a_{j,0} + \sum_i a_{j,i} \, S_i \quad (3)$$

$a_{j,i}$ est un coefficient de pondération associé à l'espèce $x_j$ pour la température $T_i$.

Dans cet exemple,

$$x_1 = f_1(S_1, S_2) = a_{1,0} + a_{1,1}S_1 + a_{1,2}S_2 \quad (4)$$

et

$$x_2 = f_2(S_1, S_2) = a_{2,0} + a_{2,1}S_1 + a_{2,2}S_2 \quad (5)$$

Les coefficients $a_{j,0}$ et $a_{j,i}$ sont préalablement définis, au cours d'une étape de calibration préalablement établie, faisant l'objet des étapes 90 à 94.

Etape 90 : Au cours de cette étape, on met en oeuvre un capteur identique ou considéré comme représentatif du capteur mis en oeuvre dans les étapes 100 et 110. Le capteur est exposé à plusieurs couples de concentrations $X_k$ connues de chaque espèce gazeuse avec $X_k = (x_{1,k}, x_{2,k})$, où $k$ désigne un rang assigné à chaque couple de concentrations $X_k$.

Etape 91 : l'élément sensible est successivement porté à chaque température $T_i$. On mesure ensuite la température de l'élément sensible Le capteur de gaz génère un signal de détection $S_{i,k}$ représentatif de la conductivité thermique $\lambda_i$ du gaz à la température $T_i$ pour le couple de concentrations connues $X_k = (x_{1,k}, x_{2,k})$ et pour la température $T_i$.

Etape 92 : l'étape 91 est répétée pour les deux températures différentes.

Etape 93 : les étapes 90 à 92 sont répétées pour les trois couples de concentrations d'espèces gazeuses : $1 \leq k \leq 3$ .

Etape 94 : A partir des signaux de détection $S_{i,k}$ résultant des trois étapes 91 successivement répétées, on détermine les facteurs de pondération $a_{j,0}$, $a_{j,i}$ précédemment définis.

Une matrice de passage $M$ est formée à partir des signaux de détection $S_{i,k}$ $M$ est telle que

$$M = \begin{bmatrix} 1 & S_{1,1} & S_{2,1} \\ 1 & S_{1,2} & S_{2,2} \\ 1 & S_{1,3} & S_{2,3} \end{bmatrix}$$

La matrice $M$ est connue car elle résulte de la mise en oeuvre du capteur.

On forme, pour chaque espèce gazeuse $x_j$, un vecteur de concentrations $V_j$, dont chaque terme est une concentration de l'espèce gazeuse dans chaque couple de concentrations.

$$V_1 = \begin{bmatrix} x_{1,1} \\ x_{1,2} \\ x_{1,3} \end{bmatrix}$$

et

$$V_2 = \begin{bmatrix} x_{2,1} \\ x_{2,2} \\ x_{2,3} \end{bmatrix}$$

Ces vecteurs de concentrations $V_j$ sont des données d'entrée. Chaque terme $x_{j,k}$ est connu. On forme un

premier vecteur $A_1$ et un deuxième vecteur $A_2$ de facteurs de pondération des fonctions de calibration respectivement associées à la première espèce gazeuse et à la deuxième espèce gazeuse.

$$A_1 = \begin{bmatrix} a_{1,0} \\ a_{1,1} \\ a_{1,2} \end{bmatrix}$$

$$A_2 = \begin{bmatrix} a_{2,0} \\ a_{2,1} \\ a_{2,2} \end{bmatrix}$$

Les vecteurs $A_1$ et $A_2$ sont inconnus.

Compte tenu de (4) et de (5) :

$$V_1 = M \times A_1 \ (7)$$

et

$$V_2 = M \times A_2 \ (8)$$

Il vient :

$$A_1 = M^{-1} \times V_1 \ (9)$$

et

$$A_2 = M^{-1} \times V_2 \ (10)$$

Les inversions (9) et (10) sont calculées lorsque la matrice $M$ est inversible, où sont obtenues sur la base d'un algorithme d'inversion dans le cas contraire. On obtient ainsi une estimation des vecteurs $A_1$ et $A_2$.

Les couples de concentrations $(x_{1,k}, x_{2,k})$ définissent, pour chaque espèce gazeuse, une concentration minimale et une concentration maximale. De préférence, la concentration minimale et la concentration maximale sont aussi éloignées que possible. La plage comprise entre la concentration minimale et la concentration maximale définit une plage de validité des fonctions de calibration respectivement associées à chaque espèce gazeuse.

Suite à l'étape 94, on dispose des facteurs de pondération définissant la fonction de calibration associée à chaque espèce gazeuse.

Selon une variante, l'expression (5) peut être remplacée par

$$x_2 = f_2(S_1, S_2) = a_{2,0} + a_{2,1}S_1 (11)$$

Auquel cas au cours de l'étape 94, on forme une matrice réduite $L$, telle que

$$L = \begin{bmatrix} 1 & S_{2,1} \\ 1 & S_{2,2} \end{bmatrix}$$

Le vecteur $A_2$ est tel que :

$$A_2 = \begin{bmatrix} a_{2,0} \\ a_{2,1} \end{bmatrix}$$

Le vecteur $V_2$ est tel que :

$$V_2 = \begin{bmatrix} x_{2,1} \\ x_{2,2} \end{bmatrix}$$

et

$$A_2 = L^{-1} \times V_2 \ (12)$$

Dans cette variante, on met en oeuvre une fonction de calibration $f_2$ plus simple. Une telle variante peut être utilisée pour estimer la concentration d'une espèce gazeuse dans une plage de concentrations restreinte préalablement déterminée.

La méthode précédemment décrite peut être étendue à la mesure de $n$ espèces gazeuses différentes, avec $n \geq 2$. Les étapes 100 et 110 sont mises en oeuvre pour $n$ températures différentes;

- Selon le modèle précédemment explicité, chaque fonction de calibration associée à une espèce comporte $n+1$ facteurs de pondération.
- Les étapes 90 à 92 sont réitérées $n + 1$ fois, pour $n+1$ couples de concentrations $X_k = (x_{j=1,k} \ldots x_{j=n,k})$ de chaque espèce gazeuse, et pour $n$ températures différentes à chaque itération.
- Au cours de l'étape 94 :

  - La matrice M est carrée est a une dimension de ($n+1 \times n+1$). Elle comporte chaque signal de détection $S_{i,k}$ avec $1 \leq i \leq n$ et $1 \leq k \leq n + 1$. La matrice comporte également n+1 termes unités.
  - On forme $n$ vecteurs $V_j$ respectivement associés à chaque espèce gazeuse $x_j$. Chaque vecteur comporte les $n+1$ concentrations $x_{j,k}$ de l'espèce gazeuse $x_j$ dans le couple de concentrations de rang $k$.
  - On prend en compte $n$ vecteurs de facteurs de pondération $A_j$ respectivement associés à chaque espèce gazeuse $x_j$.
  - Chaque vecteur $A_j$ est estimé selon $A_j = M^{-1} \times V_j$ (13)

Lorsque la fonction de calibration fait intervenir un nombre différents de facteurs de pondération, la dimension de la matrice $M$ est ajustée en conséquence. Ainsi, si la fonction de calibration fait intervenir n+2 termes, la dimension de la matrice M est n+2 $\times$ n+2. Un exemple de telle fonction de calibration est :

$$x_i = f_i(S_1, S_2) = a_{i,0} + a_{i,1}S_1 + a_{i,2}(S_1)^2 + a_{i,3}S_2 \ (14)$$

Essais expérimentaux

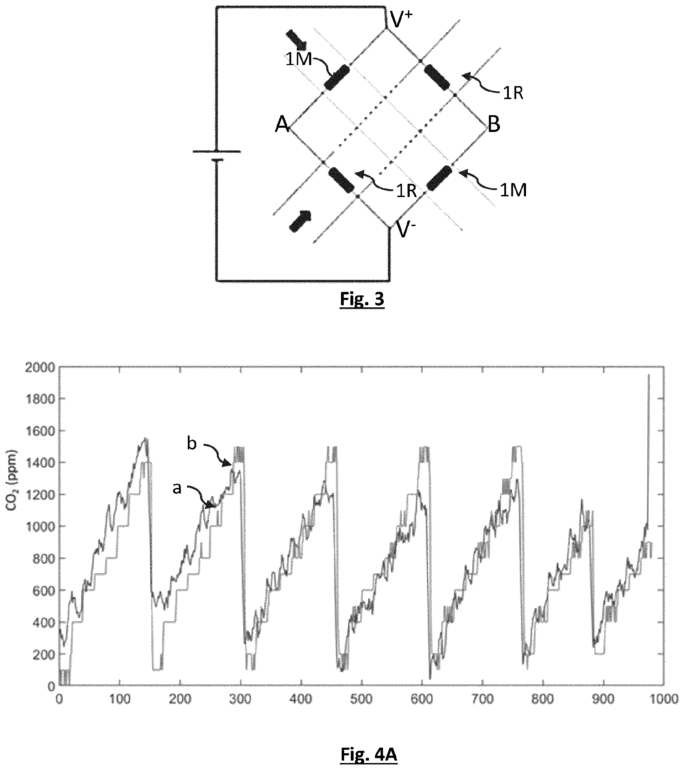

Les inventeurs ont disposé quatre capteurs 1 tels que précédemment décrits, les membranes de chaque capteur étaient montées selon un pont de Wheatstone. Cf. figure 3. Deux capteurs de référence 1R étaient exposés à de l'air, tandis que deux capteurs 1M étaient destinés à mesurer l'humidité et le $CO_2$ dans de l'air, la concentration de $CO_2$ et l'humidité relative étant variables. Les paramètres de chaque capteur sont les suivants :

- Taille de chaque membrane : 100 $\mu$m $\times$ 100 $\mu$m.
- Résistance de chaque membrane : de l'ordre de 100 à 1000 Ohms.
- Tension d'entrée sur le pont de Wheatstone : $E_1$ = 5 V ; $E_2$ = 8 V. Sur la figure 3, la tension d'entrée correspond à la tension appliquée entre les points V$^+$ et V. L'application successive de deux tensions d'entrée différentes permet d'atteindre deux températures différentes.
- Tension de sortie : 10 $\mu$V pour 100 ppm de concentration en $CO_2$ : il s'agit de la tension entre les points A et B, à un gain d'amplification près. La tension de sortie correspond au signal de détection. Elle est représentative de la quantité des espèces gazeuses recherchées ($CO_2$, humidité) dans le gaz analysé. La tension de sortie, pour une température déterminée $T_i$, forme le signal de détection $S_i$.

On a procédé à une calibration en prenant les trois couples de concentrations suivants, pour $CO_2$ (ppm) et $H_2O$ en phase vapeur (% d'humidité relative) : (400 ppm ; 60,75 %) - ( 1000 ppm ; 16.29 %) ; (400 ppm ; 5.56 %).

Au cours des mesures, on a fait varier la concentration de $CO_2$ et de vapeur d'eau dans l'air ambiant. Sur un des capteurs de mesure, on a mis en oeuvre le procédé précédemment décrit pour estimer simultanément la concentration en $CO_2$ et en vapeur d'eau de l'air ambiant. Les mesures ont été comparées avec des mesures de réalité terrain. Pour le $CO_2$, on a mis en oeuvre un capteur de réalité terrain optique. Pour $H_2O$, on a mis en oeuvre un capteur de réalité terrain capacitif.

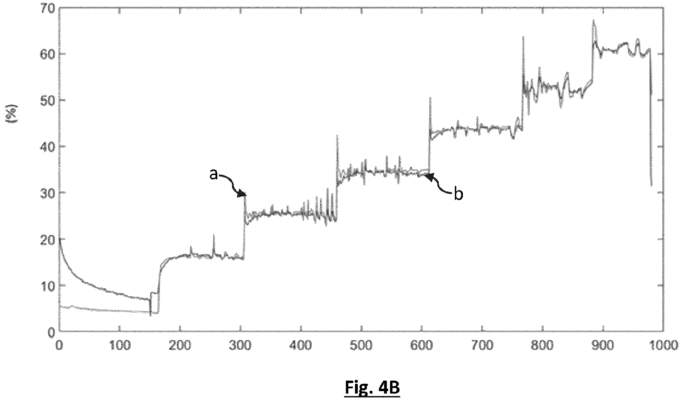

La figure 4A montre l'estimation de la concentration de $CO_2$ résultant du capteur 1M (courbe a) et du capteur de réalité terrain (courbe b). La figure 4B montre l'estimation de la concentration de vapeur d'eau résultant du capteur 1M (courbe a) et du capteur de réalité terrain (courbe b).

Sur la figure 4A chaque point est obtenu par un moyennage de 5 mesures consécutives. L'axe des abscisses correspond à chaque point de mesure et l'axe des ordonnées correspond à la concentration de $CO_2$ mesurée (unité ppm).

Sur la figure 4B, l'axe des abscisses correspond à chaque point de mesure et l'axe des ordonnées correspond à l'humidité relative (%).

Dans l'exemple décrit, chaque fonction de calibration $f_j$ est une combinaison linéaire selon laquelle les signaux de détection $S_i$ respectivement mesurés à chaque température $T_i$ sont multipliés par des facteurs de pondération $a_{j,i}$. D'autres formes de fonctions de calibration sont envisageables, par exemple des fonctions polynômiales (voir par exemple (14)), selon lesquelles au moins un signal de détection $S_i$ peut être affecté d'un exposant, par exemple $S_i^2$.

L'invention pourra être utilisée pour former des capteurs à bas coût, par exemple pour l'habitat, des véhicules, des lieux publics fermés, ou l'air extérieur. La simplicité du traitement des signaux de détection le rend compatible avec une mise en oeuvre par un dispositif nomade compact de type téléphone ou montre.

## Revendications

1. Procédé d'estimation d'une concentration de $n$ espèces gazeuses dans un gaz (3), $n$ étant un entier supérieur ou égal à 2, le procédé mettant en oeuvre un capteur de gaz (1), le capteur de gaz :

   - comportant un élément sensible (4), disposé au contact du gaz, et configuré pour être chauffé par une piste conductrice de chauffage (5), la température ($T_i$) de l'élément sensible dépendant de la conductivité thermique du gaz et d'un courant électrique de chauffage traversant la piste conductrice de chauffage;
   - comportant un circuit de mesure (6), générant un signal de détection ($S_i$) dépendant d'une température ($T_i$) de l'élément sensible;

   le procédé comportant les étapes suivantes :

   - a) exposition de l'élément sensible au gaz et chauffage de l'élément sensible de façon à atteindre $n$ différentes températures ($T_i$);
   - b) pour chaque température ($T_i$), mesure d'un signal de détection ($S_i$) et transmission du signal de détection à une unité de traitement (10);
   - c) à partir des signaux de détection respectivement obtenus à chaque température ($T_i$), estimation d'une concentration en chaque espèce gazeuse ($x_j$);

   le procédé étant tel que

   - l'étape c) comporte, pour chaque espèce gazeuse ($x_j$) une application d'une fonction de calibration ($f_j$), la fonction de calibration comportant une combinaison linéaire de termes, chaque terme dépendant du signal de détection respectivement mesuré à chaque température ($T_i$), chaque terme étant pondéré par un facteur de pondération ($a_{j,i}$);
   - chaque facteur de pondération est préalablement déterminé au cours d'une phase de calibration, au cours de laquelle le capteur de gaz, ou un capteur de référence, représentatif du capteur de gaz, est successivement exposé à $n+1$ ensembles de concentrations connues de chaque espèce gazeuse, l'élément sensible étant successivement porté aux $n$ températures lors de chaque exposition ;

   le procédé étant **caractérisé en ce que** la phase de calibration comporte :

   - i) obtention d'un signal de détection ($S_i$) lors de chaque exposition du capteur de gaz à un ensemble de concentrations connues de chaque espèce gazeuse ;
   - ii) constitution d'une matrice ($M$) à l'aide des signaux de détection obtenus lors de l'étape i) ;
   - iii) pour chaque espèce gazeuse ($x_j$), formation d'un vecteur ($V_j$), comportant les concentrations de l'espèce gazeuse dans chacun des $n+1$ ensembles de concentrations ;

- iv) calcul ou estimation d'un vecteur ($A_j$), de facteurs de pondération associés à chaque espèce gazeuse ($x_j$), ledit vecteur correspondant à un produit matriciel entre l'inverse de la matrice résultant de l'étape ii) et le vecteur résultant de l'étape iii).

**2.** Procédé selon la revendication 1, dans lequel *n* est égal à 2.

**3.** Procédé selon la revendication 2, dans lequel le gaz est de l'air ambiant, les espèces gazeuses mesurées étant le $CO_2$ et l'humidité.

**4.** Procédé selon l'une quelconque des revendications précédentes, dans lequel :

- au cours de la phase de calibration, la concentration de chaque espèce gazeuse varie entre une concentration minimale et une concentration maximale ;
- la fonction de calibration définie pour chaque espèce gazeuse est valide entre ladite concentration minimale et ladite concentration maximale.

**5.** Capteur de gaz (1), comportant :

- un élément sensible (4) configuré pour être disposé au contact du gaz, et pour être chauffé par une piste conductrice de chauffage (5), la température ($T_i$) de l'élément sensible dépendant de la conductivité thermique du gaz et d'un courant ($E_i$) traversant la piste conductrice de chauffage;
- un circuit de mesure (6) d'une température de l'élément sensible, ladite température dépendant de la conductivité thermique du gaz ;

le capteur de gaz étant configuré pour porter l'élément sensible (4) à différentes températures et pour générer un signal de détection ($S_i$) à chaque température, le signal de détection étant représentatif de la conductivité thermique du gaz à ladite température ;

le capteur de gaz comportant une unité de traitement (10), configurée pour mettre en oeuvre l'étape c) d'un procédé selon l'une quelconque des revendications précédentes.

**6.** Capteur de gaz selon la revendication 5, dans lequel l'élément sensible est une membrane.

**7.** Capteur de gaz selon l'une quelconque des revendications 5 ou 6, dans lequel la piste conductrice de chauffage est un fil électriquement conducteur.

**8.** Capteur de gaz selon l'une quelconque des revendications 5 à 7, dans lequel le circuit de mesure (6) de la température de l'élément sensible est un circuit de mesure de la résistance d'une piste électrique de détection traversant l'élément sensible.

**9.** Système de mesure comportant plusieurs capteurs de gaz selon l'une quelconque des revendications 5 à 8.

**10.** Système de mesure dans lequel au moins un capteur de gaz est exposé à un gaz de référence, dont la composition est connue, et au moins un capteur de gaz est exposé à un gaz dont la teneur en au moins une espèce gazeuse est inconnue.

**11.** Système de mesure selon la revendication 10, comportant quatre capteurs de gaz, dont au moins un capteur de gaz selon l'une quelconque des revendications 5 à 8, les capteurs de gaz étant agencés en pont de Wheatstone, de telle sorte que deux capteurs opposés sont exposés au gaz de référence et deux autres capteurs opposés sont exposés au gaz dont la teneur en au moins une espèce gazeuse est inconnue.

1

2

3

4 5

6

10

**Fig. 1**

| 90 |
| 91 |
| 92 |
| 93 |
| 94 |

| 100 |
| 110 |
| 120 |
| 130 |

**Fig. 2**

**Fig. 3**

**Fig. 4A**

**Fig. 4B**

Europäisches
Patentamt

European
Patent Office

Office européen
des brevets

**RAPPORT DE RECHERCHE EUROPEENNE**

Numéro de la demande

**EP 23 20 7815**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | UDINA S ET AL: "A micromachined thermoelectric sensor for natural gas analysis: Multivariate calibration results", SENSORS AND ACTUATORS B: CHEMICAL, ELSEVIER BV, NL, vol. 166, 29 novembre 2011 (2011-11-29), pages 338-348, XP028486933, ISSN: 0925-4005, DOI: 10.1016/J.SNB.2011.11.086 [extrait le 2012-03-01] * Sections 2, 3, 3.1, 3.2 et 3.3; pages 339-342; figures 1,2; tableau 1 * | 1-11 | INV. G01N33/00 G01N27/18 G01N25/18 |
| A | US 4 164 862 A (JACKSON MILTON L) 21 août 1979 (1979-08-21) * colonne 2, ligne 5 - colonne 7, ligne 26; figure 1 * | 1-11 | |
| A | DE 199 49 327 A1 (GRUNEWALD AXEL ULRICH [DE]) 19 avril 2001 (2001-04-19) * abrégé; revendication 1; figure 1 * | 1-11 | DOMAINES TECHNIQUES RECHERCHES (IPC) |
| A | EP 0 285 833 A2 (HARTMANN & BRAUN AG [DE]) 12 octobre 1988 (1988-10-12) * abrégé; revendications 1,4; figures 1,2 * | 1-11 | G01N |

Le présent rapport a été établi pour toutes les revendications

| Lieu de la recherche | Date d'achèvement de la recherche | Examinateur |
|---|---|---|
| La Haye | 15 février 2024 | Gilow, Christoph |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 23 20 7815

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

15-02-2024

| Document brevet cité au rapport de recherche | | Date de publication | Membre(s) de la famille de brevet(s) | | Date de publication |
|---|---|---|---|---|---|
| US 4164862 | A | 21-08-1979 | AUCUN | | |
| DE 19949327 | A1 | 19-04-2001 | DE | 19949327 A1 | 19-04-2001 |
| | | | EP | 1222454 A1 | 17-07-2002 |
| | | | US | 6688159 B1 | 10-02-2004 |
| | | | WO | 0127604 A1 | 19-04-2001 |
| EP 0285833 | A2 | 12-10-1988 | CN | 88101718 A | 26-10-1988 |
| | | | DE | 3711511 C1 | 30-06-1988 |
| | | | EP | 0285833 A2 | 12-10-1988 |
| | | | US | 4902138 A | 20-02-1990 |

EPO FORM P0460

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 3118611 B1

**Littérature non-brevet citée dans la description**

- **UDINA S et al.** *A micromachined thermoelectric sensor for natural gas analysis : Multivariate calibration results*